# EUROPEAN PATENT SPECIFICATION

(11) **EP 3 983 070 B1**
(45) Date of publication and mention of the grant of the patent: **11.09.2024**
(21) Application number: 20826406.9
(22) Date of filing: 15.06.2020
(51) Int. Cl.: A61K 31/275, A61P 11/06, C07C 317/26, C07C 317/28, C07C 317/48, C07C 317/50

(54) **METHOD FOR TREATING ASTHMA**
VERFAHREN ZUR BEHANDLUNG VON ASTHMA
MÉTHODE DE TRAITEMENT DE L'ASTHME

(30) Priority: 17.06.2019 US 201962862434 P
(43) Date of publication of application: 20.04.2022
(73) Proprietor: Olatec Therapeutics, Inc., New York, NY 10065 (US)
(72) Inventor: DINARELLO, Charles A., New York, New York 10065 (US); MARCHETTI, Carlo, New York, New York 10065 (US)
(74) Representative: Schüssler, Andrea
(86) International application number: PCT/US2020/037696
(87) International publication number: WO 2020/257093

(56) References cited:
- WO-A1-2017/129897
- US-A1- 2019 119 203
- YANG YANG ET AL: "Recent advances in the mechanisms of NLRP3 inflammasome activation and its inhibitors", CELL DEATH & DISEASE, vol. 10, no. 2, 1 February 2019 (2019-02-01), XP055626877, DOI: 10.1038/s41419-019-1413-8
- KIM RICHARD Y ET AL: "Role for NLRP3 Inflammasome-mediated, IL -lb-Dependent Responses in Severe, Steroid -Resistant Asthma", AMERICAN JOURNAL OF RESPIRATORY AND CRITICAL CARE MEDICINE, AMERICAN THORACIC SOCIETY, US, vol. 196, no. 3, 1 August 2017 (2017-08-01), pages 283 - 297, XP009524669, ISSN: 1073-449X, DOI: 10.1164/RCCM.201609-1830OC
- CHEN SHUYU ET AL: "Blockade of the NLRP3/Caspase-1 Axis Ameliorates Airway Neutrophilic Inflammation in a Toluene Diisocyanate-Induced Murine Asthma Model", TOXICOLOGICAL SCIENCES, vol. 170, no. 2, 17 April 2019 (2019-04-17), pages 462 - 475, XP093031760, ISSN: 1096-6080, Retrieved from the Internet <URL:https://watermark.silverchair.com/kfz099.pdf?token=AQECAHi208BE49Ooan9kkhW_Ercy7Dm3ZL_9Cf3qfKAc485ysgAAAt0wggLZBgkqhkiG9w0BBwagggLKMIICxgIBADCCAr8GCSqGSIb3DQEHATAeBglghkgBZQMEAS4wEQQMqBVdr_y9YOkLpAYvAgEQgIICkMt67Gd12QQfxDQ49eecttui1KCfqaHVI8XwogIHMwk14HMic7KVWj_sFuypbsIch21rSGRCYo2MITWauivqZFDgUHmnG> DOI: 10.1093/toxsci/kfz099
- KIM RICHARD Y ET AL.: "Role for NLRP3 inflammasome-mediated, IL-1 fl-dependent responses insevere, steroid-resistant asthma: Online Supplement", AMERICAN JOURNAL OF RESPIRATORY AND CRITICAL CARE MEDICINE, vol. 196, no. 3, August 2017 (2017-08-01)
- SWANSON KAREN V ET AL: "The NLRP3 inflammasome: molecular activation and regulation to therapeutics", NATURE REVIEWS IMMUNOLOGY, NATURE PUBLISHING GROUP UK, LONDON, vol. 19, no. 8, 29 April 2019 (2019-04-29), pages 477 - 489, XP036846104, ISSN: 1474-1733, [retrieved on 20190429], DOI: 10.1038/S41577-019-0165-0
- MARCHETTI ET AL.: "OLT1177, a beta-sulfonyl nitrile compound, safe in humans, inhibits the NLRP3 inflammasome and reverses the metabolic cost of inflammation", PNAS, vol. 115, no. 7, 29 January 2018 (2018-01-29), pages E1530 - E1539, XP055518515, DOI: 10.1073/pnas.1716095115

## Description

### FIELD OF THE INVENTION

The present invention relates to 3-methanesulfonylpropionitrile (dapansutrile), or its pharmaceutically acceptable solvates, for use in a method of treating asthma.

### BACKGROUND

Asthma is a common chronic disorder of the airways characterized by variable and recurring symptoms, reversible airway obstruction, bronchial hyperresponsiveness, and an underlying inflammation. Acute symptoms of asthma include cough, wheezing, shortness of breath and nocturnal awakening. Asthma is regarded as a chronic disease based on a condition of chronic airway inflammation together with airway hyperresponsiveness, with at least partially reversible airway obstruction.

Central to the pathophysiology of asthma is the presence of underlying airway inflammation mediated by the recruitment and activation of multiple cell types including mast cells, eosinophils, T lymphocytes, macrophages, dendritic cells and neutrophils. Type 2 T-helper (Th2) cells appear to play a central role in the activation of the immune cascade that results in inflammation. Th2-derived cytokines include IL-5, which is needed for eosinophil differentiation and survival, and IL-4 which is important for Th2 cell differentiation and with IL-13 is important for IgE formation and leads to overproduction of IgE and eosinophilia. IgE-driven activation of mucosal mast cells releases bronchoconstrictor mediators such as histamine and cysteinyl-leukotrienes as well as inflammatory cytokines. Eosinophils contain inflammatory enzymes, generate leukotrienes, and express a wide variety of proinflammatory cytokines. Airway epithelial cells also play a role in the inflammatory process via release of cytokines such as eotaxin that direct and modify the inflammatory response. Acute and chronic inflammation can affect not only the airway caliber and airflow but also can increase the existing bronchial hyperresponsiveness to a variety of stimuli, which enhances susceptibility to bronchospasm.

As a consequence of airway inflammation and the generation of growth factors, the airway smooth muscle cell can undergo proliferation, activation, contraction, and hypertrophy; which are events that can influence airway airflow limitation. In asthma, the dominant physiological event leading to clinical symptoms is airway narrowing and a subsequent interference with airflow. In acute exacerbations of asthma, bronchial smooth muscle contraction (bronchoconstriction) occurs quickly to narrow the airways in response to exposure to a variety of stimuli including allergens or irritants. Allergen-induced acute bronchoconstriction results from an IgE-dependent release of mediators from mast cells that includes histamine, tryptase, leukotrienes, and prostaglandins that directly contract airway smooth muscle. The mechanisms influencing airway hyperresponsiveness are multiple and they include inflammation, dysfunctional neuroregulation, and airway remodeling. Airway remodeling involves structural changes such as thickening of the sub-basement membrane, subepithelial fibrosis, airway smooth muscle hypertrophy and hyperplasia, blood vessel proliferation and dilation with consequent permanent changes in the airway that increase airflow obstruction.

Airway epithelium and endothelial cell function are also critically involved in asthma. Upon disease progression, epithelial subbasement membranes thicken with mucus hypersecretion and the formation of mucus plugs. Changes in endothelial cell integrity lead to edema, another key pathophysiology defining asthmatic change of the airway. These factors serve to further limit airflow.

Asthma is characterized by dominant T helper type 2 (Th2) immune responses, including enhanced IL-4, IL-5 and IL-13 responses, allergen-specific immunoglobulin production, eosinophilia, airway inflammation, bronchoconstriction, and airway hyperresponsiveness. Current standard therapies for asthma are a combination of corticosteroids and β₂-agonists (anti-inflammatory and bronchodilator drugs). These drugs provide acceptable control of the disease for many asthmatics. However, it is estimated that 5 to 10% of the asthma patients have symptomatic disease despite treatment with this combination of corticosteroids and β₂-agonists.

Kim et al. (American Journal of Respiratory and Critical Care Medicine, Vol. 196, No. 3, pp. 283-297, 2017) discloses the role for NLRP3 Inflammasome-mediated, IL-1b-Dependent Responses in Severe, Steroid-Resistant Asthma.

Chen et al. (Toxicological Sciences, 170(2), pp. 462-475, 2019) describes that the blockade of the NLRP3/Caspase-1 Axis ameliorates airway neutrophilic inflammation in a toluene diisocyanate-induced murine asthma model.

There is a need to develop a new method for effectively treating asthma. The method should be effective with minimal side effects.

### BRIEF DESCRIPTION OF THE DRAWINGS

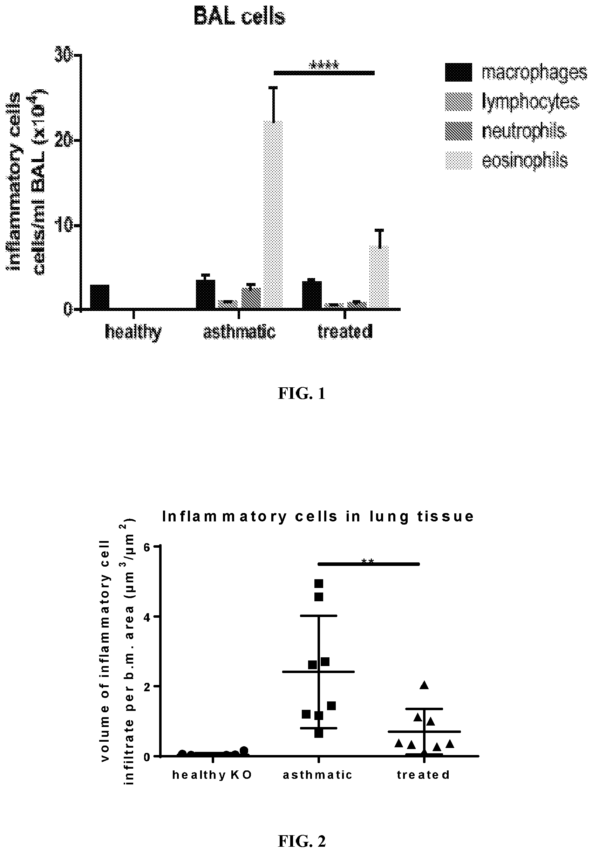

FIG. 1 shows total numbers (mean ± SEM) of leukocyte subpopulations (macrophage, lymphocyte, neutrophil, and eosinophil, from left to right) in bronchoalveolar lavage fluids on Day 29, 24 hours after the final ovalbumin (OVA) aerosol challenge and intraperitoneal dapansutrile dose. ****p < 0.0001, eosinophil, between asthmatic and treated mice (n=8 per group). Mice were treated with 60 mg/kg dapansutrile intraperitoneally.
FIG. 2 shows volume of inflammatory cells infiltrate per basal membrane in lung tissue of mice treated with dapansutrile. **p < 0.01 between asthmatic and treated mice (n=8 per group). Mice were treated with 60 mg/kg dapansutrile intraperitoneally.

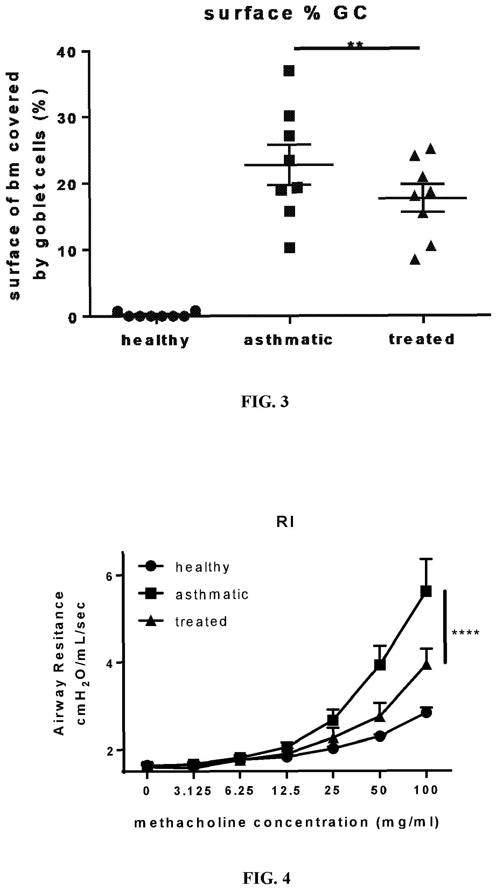

FIG. 3 shows area of epithelial basal membrane (mean ± SEM) covered by goblet cells in healthy, asthmatic, and treated mice (n=8 per group). **p < 0.01 between asthmatic and treated mice. Mice were treated with 60 mg/kg dapansutrile intraperitoneally.
FIG. 4 shows mean ± SEM airway resistance toward methylcholine for in healthy, asthmatic, and dapansutrile-treated mice. ****p < 0.0001 between asthmatic and treated mice. MCh (acetyl-β-methylcholine chloride) provocation testing started with PBS, followed by MCh aerosols with increasing concentrations from 0 to 100 mg/mL (n=8 per group). Mice were treated with 60 mg/kg dapansutrile intraperitoneally.

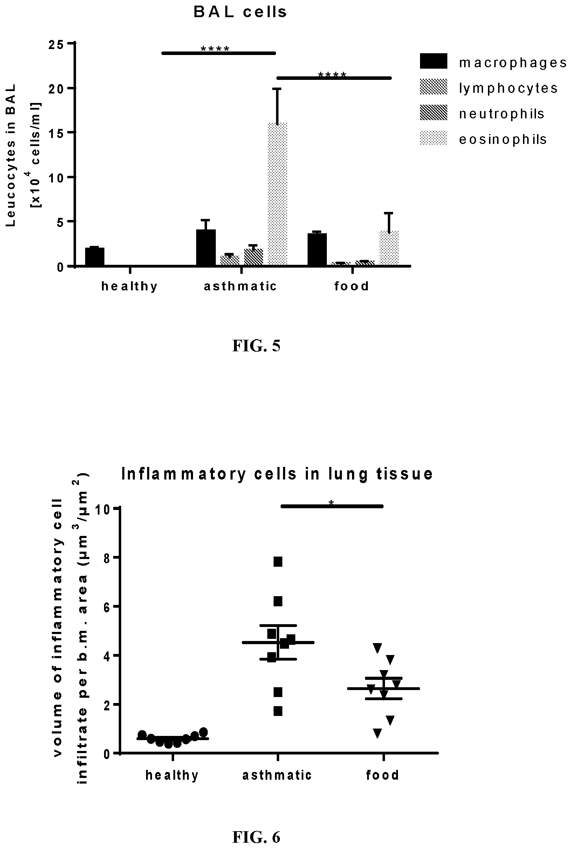

FIG. 5 shows total numbers of leukocyte subpopulations (macrophage, lymphocyte, neutrophil, and eosinophil, from left to right, mean ± SEM) in bronchoalveolar lavage fluids on Day 29, 24 hours after the final ovalbumin (OVA) aerosol challenge and oral dapansutrile dose. ****p < 0.0001, eosinophil, between asthmatic and treated mice (n=8 per group).
FIG. 6 shows volume of inflammatory cells infiltrate per basal membrane in lung tissue of mice orally treated with dapansutrile. *p < 0.05 between asthmatic and treated mice (n=8 per group).

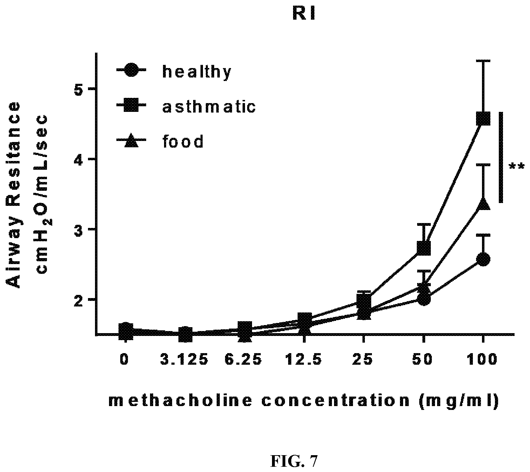

FIG. 7 shows mean ± SEM airway resistance toward methylcholine for in healthy, asthmatic, and dapansutrile-treated mice. **p < 0.01 between asthmatic and treated mice. MCh (acetyl-β-methylcholine chloride) provocation testing started with PBS, followed by MCh aerosols with increasing concentrations from 0 to 100 mg/mL (n=8 per group). Mice were orally treated with dapansutrile in feed at 7.5 g/kg food.

### DETAILED DESCRIPTION OF THE INVENTION

The inventor has discovered that 3-methanesulfonylpropionitrile, which reduces the levels of IL-1β and IL-6 in several whole animal models of local and systemic inflammation, is effective in treating asthma, reducing airway inflammation, reducing airway resistance, improving lung function, ameliorating asthma symptoms, and improving patient's quality of life.

The present invention concerns the field of asthma treatment. The present invention is directed to a compound of 3-methanesulfonylpropionitrile (dapansutrile), or a pharmaceutically acceptable solvate thereof, for use in a method of treating asthma by oral administration of said compound.

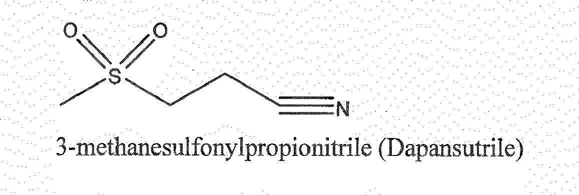

"Solvates," as used herein, are addition complexes in which the compound is combined with an acceptable solvent in some fixed proportion. Acceptable solvents include, but are not limited to, water, acetic acid, ethanol, and other appropriate organic solvents.

In one embodiment, the present method is effective in prophylactic treatment, which is a process of protecting against the development of asthma by a treatment of dapansutrile before the onset of asthma to affect pathogenesis. By prophylactic treatment, dapansutrile is administered to a patient in need thereof, before the onset of asthma.

In another embodiment, the present method is effective in therapeutic treatment after the onset of asthma, when the patient starts to show clinical signs and/or symptoms.

The main functional changes of the lungs associated asthma include malfunctioning of the immune system, cellular infiltration composed primarily of eosinophils and neutrophils, acute and chronic inflammation, fluid accumulation (edema), excessive secretion of mucus, and changes in the airway walls that could lead to bronchial epithelial injury, fibrosis, and increased sensitivity to agents that cause bronchial constriction. These features need to be considered in order to develop treatments of the underlying disease process. Small animal models can be designed to mimic the airway inflammation, increased responsiveness to bronchial constrictors, changes in the airway wall, and changes in the migration of the eosinophils and neutrophils to the lungs. A mouse model of asthma via ovalbumin sensitization (Lunding, 2015b), for example, can be used to evaluate bronchodilator efficacy of dapansutrile.

The present invention is based on the properties of dapansutrile to reduce at least one of the following processes contributing to pathophysiology that accompanies this disorder: inflammation, excessive cell proliferation, airway and/or lung tissue edema, airway hyperreactivity, and bronchoconstriction.

Indicia of efficacy for treating asthma by the present invention include demonstrable improvement in measurable signs, symptoms and other variables clinically relevant to asthma. Such improvements include increased blood oxygen saturation, decreased hypoxia and hypercapnia, decreased need for supplemental oxygen, decreased frequency of coughing and/or wheezing, improved forced expiratory volume in one second (FEVi), improved forced vital capacity (FVC) or other physiologically relevant parameter of respiratory function, decreased need for mechanical ventilation, decreased amount of inflammatory cells infiltrating the lung, decreased levels of proinflammatory cytokines and chemokines, improved alveolar fluid clearance rate, decreased pulmonary edema as determined by any radiographic or other detection method such as amount of epithelial lining fluid, wet to dry lung weight, alveolar fluid clearance and/or radiographic visualization methods, increase in general quality of life, patient-reported or physician-observed signs such as ease of breathing, or decrease in severity of coughing and/or wheezing.

The present invention treats asthma by (i) improving symptoms (daytime and nocturnal symptoms, limitation of activities, use of rescue medications), (ii) improving lung function such as peak expiratory flow (PEF) and/or forced expiratory volume in one second (FEV1), and/or (iii) reducing exacerbations (rate and severity). The present invention improves Asthma Quality of Life Questionnaire (AQLQ) scores, which include the scores of symptoms, activity limitation, emotional function, and environmental exposure.

The present invention has demonstrated that dapansutrile reduced airway resistance, reduced inflammatory cells (eosinophils and neutrophils) and mucus hyperproduction in broncho-alveolar lavage fluid, and reduced airway inflammation, in ovalbumin-induced allergic airway inflammation in mice.

### Pharmaceutical Compositions

The present invention provides pharmaceutical compositions comprising one or more pharmaceutically acceptable carriers and an active compound of 3-methanesulfonylpropionitrile, or a pharmaceutically acceptable solvate thereof. The active compound or its pharmaceutically acceptable solvate in the pharmaceutical compositions in general is about 1-90% for a tablet formulation or about 1-100% for a capsule formulation. The active compound used in the pharmaceutical composition in general is at least 90%, preferably 95%, or 98%, or 99% (w/w) pure.

In one embodiment, the pharmaceutical composition is in a dosage form such as tablets, capsules, granules, fine granules, powders or syrups suitable for oral administration.

Pharmaceutically acceptable carriers, which are inactive ingredients, can be selected by those skilled in the art using conventional criteria. Pharmaceutically acceptable carriers include, but are not limited to, non-aqueous based solutions, suspensions, emulsions, microemulsions, micellar solutions, gels, and ointments. The pharmaceutically acceptable carriers may also contain ingredients that include, but are not limited to, saline and aqueous electrolyte solutions; ionic and nonionic osmotic agents such as sodium chloride, potassium chloride, glycerol, and dextrose; pH adjusters and buffers such as salts of hydroxide, phosphate, citrate, acetate, borate; and trolamine; antioxidants such as salts, acids and/or bases of bisulfite, sulfite, metabisulfite, thiosulfite, ascorbic acid, acetyl cysteine, cysteine, glutathione, butylated hydroxyanisole, butylated hydroxytoluene, tocopherols, and ascorbyl palmitate; surfactants such as lecithin, phospholipids, including but not limited to phosphatidylcholine, phosphatidylethanolamine and phosphatidyl inositiol; poloxamers and poloxamines, polysorbates such as polysorbate 80, polysorbate 60, and polysorbate 20, polyethers such as polyethylene glycols and polypropylene glycols; polyvinyls such as polyvinyl alcohol and povidone; cellulose derivatives such as methylcellulose, hydroxypropyl cellulose, hydroxyethyl cellulose, carboxymethyl cellulose and hydroxypropyl methylcellulose and their salts; petroleum derivatives such as mineral oil and white petrolatum; fats such as lanolin, peanut oil, palm oil, soybean oil; mono-, di-, and triglycerides; polymers of acrylic acid such as carboxypolymethylene gel, and hydrophobically modified cross-linked acrylate copolymer; polysaccharides such as dextrans and glycosaminoglycans such as sodium hyaluronate. Such pharmaceutically acceptable carriers may be preserved against bacterial contamination using well-known preservatives, these include, but are not limited to, benzalkonium chloride, ethylenediaminetetraacetic acid and its salts, benzethonium chloride, chlorhexidine, chlorobutanol, methylparaben, thimerosal, and phenylethyl alcohol, or may be formulated as a non-preserved formulation for either single or multiple use.

For example, a tablet formulation or a capsule formulation of the active compound may contain other excipients that have no bioactivity and no reaction with the active compound. Excipients of a tablet may include fillers, binders, lubricants and glidants, disintegrators, wetting agents, and release rate modifiers. Binders promote the adhesion of particles of the formulation and are important for a tablet formulation. Examples of binders include, but not limited to, carboxymethylcellulose, cellulose, ethylcellulose, hydroxypropylmethylcellulose, methylcellulose, karaya gum, starch, starch, and tragacanth gum, poly(acrylic acid), and polyvinylpyrrolidone.

### Method of Administration

The present invention is directed to dapansutrile for use in a method of treating Asthma. The method comprises the steps of first identifying a subject suffering from asthma or has a propensity to develop asthma, and administering to the subject the active compound dapansutrile, in an amount effective to treat asthma. "An effective amount," as used herein, is the amount effective to treat asthma by ameliorating the pathological condition, reducing airway inflammation, reducing airway hyperresponsiveness, improving lung function, and/or reducing the symptoms of asthma.

An oral administration is suitable for the present invention.

In such an administration, the active compound first reaches plasma and then distributes into target tissues.

According to the present invention, the pharmaceutical composition is administrated orally to the subject. The dosage for oral administration is generally at least 0.1 mg/kg/day and less than 100 mg/kg/day or 200mg/kg/day. For example, the dosage for oral administration is 1-100, or 5-50, or 10-50 mg/kg/day, for a human subject. For example, the dosage for oral administration is 100-10,000 mg/day, and preferably 500-2000, 500-4000, 500-4000, 1000-5000, 2000-5000, 2000-6000, or 2000-8000 mg/day for a human subject. The drug can be orally taken once, twice, three times, or four times a day.

The present invention is useful in treating a mammal subject, such as humans, horses, and dogs. The present invention is particularly useful in treating humans.

The following examples further illustrate the present invention. These examples are intended merely to be illustrative of the present invention and are not to be construed as being limiting.

### EXAMPLES

### Example 1 (COMPARATIVE EXAMPLE). Dapansutrile treatment (intraperitoneal) reduced allergic airway inflammation and mucus production in mice

A well-established mouse model of experimental allergic asthma (Sel 2008, Wegmann 2005, Wegmann 2007, Lunding 2015a) was used to evaluate dapansutrile as a therapeutic option in allergic bronchial asthma and to determine if dapansutrile would have an impact on airway inflammation and the development of airway hyperresponsiveness (AHR). Inflammation in this model is characterized by the infiltration of eosinophils as well as of TH2 cells and involves the subsequent development of AHR and mucus hyperproduction so that this model resembles the major pathophysiologic hallmarks of human bronchial asthma.

### Methods and Materials

C57BL/6j mice were sensitized to OVA (ovalbumin) by three intraperitoneal (i.p.) injections of 10 µg OVA adsorbed to 150 mg aluminum hydroxide on days 1, 14, and 21. This sensitization results in an adoptive immune response against OVA with OVA-specific TH2 cells and the production of OVA-specific antibodies of the subclasses IgE and IgG4.

To induce acute allergic airway inflammation, mice were exposed three times to an OVA aerosol (1% w/v in PBS) on days 26, 27, and 28.

Healthy control animals (healthy group) were sham sensitized to PBS and subsequently challenged with PBS aerosol. Non-drug treated animals (asthmatic group) and drug-treated animals (treated group) were sensitized by OVA aerosol and subsequently challenged with OVA aerosol. The treatment group were treated with dapansutrile at 60 mg/kg at days 25, 26, 27, and 28 by intraperitoneal (i.p). injection, whereas the healthy group and the asthmatic group were administered with saline by intraperitoneal injection on days 25, 26, 27, and 28.

Eight animals per group were used. All animals were sacrificed on day 29. The following readouts were measured according to Lunding, 2015b.
- General infiltration of inflammatory cells into the broncheoalveolar lumen
- Determination of the specific infiltration of eosinophils, neutrophils and lymphocytes by histologic differentiation of the bronchoalveolar lavage (BAL) cells.
- Goblet cell hyperplasia by CAST system (Computer Assisted Stereological Toolbox), including making microscopic pictures of the airways.
- Airway resistance in response to methacholine to determine the airway hyperresponsiveness.

Specifically, inflammatory cell subpopulations (eosinophils, macrophages, neutrophils, lymphocytes) infiltrating the bronchoalveolar lumen were quantified using cyto-spinned and quick-diff-stained cells from bronchoalveolar lavage fluids (BALF). Further, inflammatory cell infiltration was recorded from hematoxylin and eosin (HE)-stained lung/airway cross-sections. AHR was assessed by recording airway resistance during a methacholine (MCh) provocation test in mice mechanically ventilated by a Buxco FinePoint RC unit. Mucus hyperproduction was quantified in PAS (periodic acid-Schiff)-stained airway cross-sections undergoing systematic, uniform random sampling and subsequent stereologic analysis of mucus amount in the airways and mucus producing goblet cells in the airway mucosa.

### Results

Allergic airway inflammation and mucus hyperproduction were assessed on Day 29, 24 hours after the final OVA aerosol challenge and dapansutrile dose. FIG. 1 shows total numbers of leukocyte subpopulations (macrophage, lymphocyte, neutrophil, and eosinophil, from left to right) on Day 29.

Dapansutrile (60 mg/kg), administered as 4 i.p. injections one day prior and concurrently with three daily OVA aerosol challenges, led to a significant reduction in eosinophils in BALF (FIG. 1). Comparing to asthmatic mice, treatment with dapansutrile resulted in an approximately 60% reduction in eosinophils (from 21.96 × 10⁴ cells/ml to 7.30 × 10⁴ cells/ml; ****p < 0.0001), a 70% reduction in neutrophils (from 2.41 × 10⁴ cells/ml to 0.70 × 10⁴ cells/ml; p < 0.01), and a 32% reduction in lymphocytes (from 0.80 × 10⁴ cells/ml to 0.55 × 10⁴ cells/ml) in BALF. Macrophage numbers showed no significant reduction (FIG. 1).

FIG. 2 shows that the number of inflammatory cells in lung tissue was significantly lower in asthmatic mice vs. dapansutrile-treated mice (**p < 0.01). The label on the y-axis reads "volume of inflammatory cell infiltrate per basal membrane area (µm³/µm²)". The inflammatory cells were counted within a specific distance around the airways using a microscope with the computer assisted stereological toolbox (CAST) system. These counts were set in ratio to the basal membrane to normalize within each microscopic slide to avoid slide-dependent differences.

FIG. 3 shows area of epithelial basal membrane covered by goblet cells in healthy, asthmatic, and treated mice. Comparing to asthmatic mice, Dapansutrile-treated animals displayed a significant reduction of goblet cells covering the airway mucosa (22.74% reduced to 17.67%, p <0.01) as quantified by stereology of PAS-stained airway cross-sections.

The results of FIGs. 1-3 show that dapansutrile treatment in OVA-induced allergic airway inflammation model resulted in significant reduction of allergic airway inflammation and mucus production.

Airway hyperresponsiveness was determined by measuring airway resistance on Day 29 in response to 100 mg/mL methacholine. The results are shown in FIG. 4.

The airway resistance in response to methacholine was 5.61 cm H₂O·sec·ml⁻¹ in asthmatic mice and 3.93 cm H₂O·sec·ml⁻¹ in dapansutrile-treated mice. Dapansutrile treatment reduced airway resistance by about 60% when comparing with asthmatic mice. ****p < 0.0001 between asthmatic and treated mice. Baseline airway resistance of healthy animals was 2.83 cm H₂O·sec·ml⁻¹.

### Example 2 (COMPATIVE EXAMPLE). Cytokines in BALE

Cytometric bead arrays were used as a method to assess the concentration of cytokines of IFN-γ, TNFα, IL-1β, IL-4, IL-5, IL-6, IL-10, IL-13, and IL17A) in the BALF of Example 1. The beads in this array were coated with antibodies specific against a variety of cytokines, some relevant and known to be affected by NLRP3 signaling. The concentrations of all measured cytokines showed some reduction between asthmatic mice and dapansutrile-treated mice. Both IL-1β and IL-6 concentration showed a statistically significant reduction between asthmatic mice and dapansutrile-treated mice (p<0.001 and p<0.05, respectively).

### Example 3. Dapansutrile treatment (oral) reduced allergic airway inflammation and airway resistance in mice

The experimental protocols of the mouse model were the same as those described in Example 1, except dapansutrile was administered orally by food.

The already-sensitized mice were fed ad libidum with food pellets containing 7.5 g/kg dapansutrile starting on day 22 for one week; the first aerosol challenge was on day 26. Mice typically consume about 4 g of food per day, resulting in an approximate daily dose of 0 mg/kg/day for control groups and 1,000 mg/kg/day for the treatment groups. This food pellet concentration (7.5g/kg of dapansutrile in food) in mouse chow resulted in a blood level nearly the same as that of humans treated orally with dapansutrile at doses of 1000 mg/day (40 µg/mL blood level; Marchetti 2018b)

Sham-sensitized, OVA-challenged mice were used as healthy controls (healthy). OVA-sensitized, OVA-challenged asthmatic controls (asthmatic) were fed with control food pellets without dapansutrile.

Comparing to asthmatic mice, treatment with dapansutrile (food) resulted in an approximate 75% reduction in eosinophils (from 15.93 × 10⁴ cells/ml to 3.77 × 10⁴ cells/ml; p < 0.0001), an approximate 75% reduction in neutrophils (from 1.74 × 10⁴ cells/ml to 0.43 × 10⁴ cells/ml; p < 0.05), and an approximate 75% reduction in lymphocytes (from 1.00 × 10⁴ cells/ml to 0.26 × 10⁴ cells/ml, p< 0.05) in BALF. Macrophage numbers showed no significant reduction (FIG. 5).

FIG. 6 shows that the number of inflammatory cells in lung tissue was significantly lower in asthmatic mice vs. dapansutrile-treated mice (p< 0.05). The label on the y-axis reads "volume of inflammatory cell infiltrate per basal membrane area (µm³/µm²)". The inflammatory cells were counted within a specific distance around the airways using a microscope with the computer assisted stereological toolbox (CAST) system. These counts were set in ratio to the basal membrane to normalize within each microscopic slide to avoid slide-dependent differences.

Comparing to asthmatic mice, Dapansutrile-treated mice also displayed a prominent reduction of goblet cells covering the airway mucosa (-29%; 15.75% reduced to 11.16%), as quantified by stereology of PAS-stained airway cross-sections. (data not shown).

The airway resistance of dapansutrile-treated animals in response to MCh was significantly lowered and revealed reductions of approximately 60% at 100 mg/mL MCh, as compared to the sham-treated asthmatic controls (4.57 cm H₂O.s/mL reduced to 3.38 cm H₂O.s/mL) (FIG. 6). **p < 0.01 between asthmatic and treated mice. Baseline airway resistance of healthy animals was 2.58 cm H₂O.s/mL at 100 mg/mL MCh.

The results show that dapansutrile-treatment by food intake resulted in significant reduction of allergic airway inflammation.

### References:

Lunding L, Webering S, Vock C, et al. IL-37 requires IL-18Ra and SIGIRR/IL-1R8 to diminish allergic airway inflammation in mice. Allergy 2015a Apr;70(4):366-73.
Lunding LP, Webering S, Vock C, et al. Poly(inosinic-cytidylic) acid-triggered exacerbation of experimental asthma depends on IL-17A produced by NK cells. J Immunol 2015b;194:5615-5625 .
Marchetti C, Swartzwelter B, Koenders MI, et al. The NLRP3 Inflammasome Inhibitor OLT1177™ Suppresses Joint Inflammation in Murine Models of Acute Arthritis. Arthritis Research and Therapy 2018b;20:169.
Sel S, Wegmann M, Dicke T, et al. Effective prevention and therapy of experimental allergic asthma using a GATA-3-specific DNAzyme. J Allergy Clin Immunol 2008;121:910-916.e5. Wegmann M, Fehrenbach H, Held T, et al. Involvement of distal airways in a chronic model of experimental asthma. Clin Exp Allergy 2005 Oct;35(10):1263-71.
Wegmann M, Göggel R, Sel S, et al. Effects of a low-molecular-weight CCR-3 antagonist on chronic experimental asthma. Am JRespir Cell Molec Bio 2007;36(1):61-7.

The invention, and the manner and process of making and using it, are now described in such full, clear, concise and exact terms as to enable any person skilled in the art to which it pertains, to make and use the same. To particularly point out and distinctly claim the subject matter regarded as invention, the following claims conclude the specification.

## Claims

1. Dapansutrite, or a pharmaceutically acceptable solvate thereof, as a pharmaceutical compound for use in a method for treating asthma, wherein said compound is administered by oral administration.

2. The compound for the use according to Claim 1, which reduces airway inflammation, reduces airway hyperresponsiveness, improves lung function, and/or reducing the symptoms of asthma.

3. The compound for the use according to Claim 1, wherein the treating is a therapeutic treatment, and dapansutrile is administered to the subject when the subject shows clinical signs and/or symptoms of asthma.

4. The compound for the use according to Claim 1, wherein the treating is a prophylactic treatment, and dapansutrile is administered to the subject before the onset of asthma.

## Patentansprüche

1. Dapansutril oder ein pharmazeutisch verträgliches Solvat davon als eine pharmazeutische Verbindung zur Verwendung in einem Verfahren zur Behandlung von Asthma, wobei die Verbindung durch orale Gabe verabreicht wird.

2. Verbindung für die Verwendung gemäß Anspruch 1, welche Entzündungen der Luftwege reduziert, Überreaktionsfähigkeit der Luftwege reduziert, die Lungenfunktion verbessert und/oder die Symptome von Asthma reduziert.

3. Verbindung für die Verwendung gemäß Anspruch 1, wobei die Behandlung eine therapeutische Behandlung ist, und Dapansutril dem Probanden verabreicht wird, wenn der Proband klinische Zeichen und/oder Symptome von Asthma zeigt.

4. Verbindung für die Verwendung gemäß Anspruch 1, wobei die Behandlung eine prophylaktische Behandlung ist, und Dapansutril dem Probanden vor dem Ausbruch von Asthma verabreicht wird.

## Revendications

1. Dapansutrile ou solvate pharmaceutiquement acceptable de celui-ci, en tant que composé pharmaceutique destiné à être utilisé dans un procédé de traitement de l'asthme, dans lequel ledit composé est administré par voie orale.

2. Composé destiné à être utilisé selon la revendication 1, qui réduit l'inflammation des voies respiratoires, réduit l'hyperréactivité des voies respiratoires, améliore la fonction pulmonaire et/ou réduit les symptômes de l'asthme.

3. Composé destiné à être utilisé selon la revendication 1, dans lequel le traitement est un traitement thérapeutique, et le dapansutrile est administré au sujet lorsque celui-ci présente des signes cliniques et/ou des symptômes d'asthme.

4. Composé destiné à être utilisé selon la revendication 1, dans lequel le traitement est un traitement prophylactique, et le dapansutrile est administré au sujet avant l'apparition de l'asthme.
